# EUROPEAN PATENT APPLICATION

(11) **EP 1 889 830 A1**
(43) Date of publication of application: **20.02.2008**
(21) Application number: 06014302.1
(22) Date of filing: 10.07.2006
(51) Int. Cl.: C07C 233/66, C07D 213/82, A61K 31/4418

(54) **Biarylcarboxyarylamides as vanilloid-1 receptor modulators**

(71) Applicant: Pharmeste S.r.l., 44100 Ferrara (IT)
(72) Inventor: Baraldi, Pier Giovanni, 44100 Ferrara (IT); Borea, Pier Andrea, 44100 Ferrara (IT); Geppetti, Pierangelo, 44100 Ferrara (IT); Pavani, Maria Giovanna, 44100 Ferrara (IT); Fruttarolo, Francesca, 44100 Ferrara (IT); Trevisani, Marcello, 44100 Ferrara (IT)
(74) Representative: Minoja, Fabrizio

(57) **Abstract**

The present invention provides compounds of formula (I) 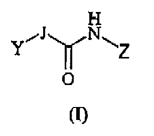 wherein Y, J and Z are as defined in the description, along with methods for preparing such derivatives and their use for the treatment of inflammatory diseases such as neuropathic pain.

## Description

### Field of the invention

The present invention relates to modulators of the vanilloid receptor, in particular to TRPV1 antagonists.

### State of the art

The Transient Receptor Potential Vanilloid 1 (TRPV1) is strongly involved in the genesis of thermal and mechanical hyperalgesia and has been proposed to play a key role in different pathological conditions including neuropathic pain and urological disorders.

Vanilloid receptor antagonists containing a biaryl moiety are known; WO 2004/056774¹ discloses, among others, the following substituted biaryl-4-carboxylic acid arylamides:

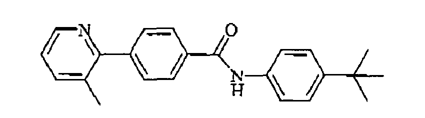

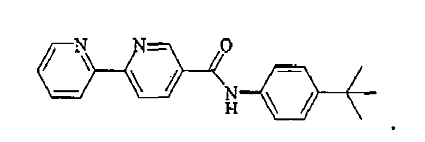

### Description of the invention

It has now been found that by replacing the pyridyl mojety in the above compounds with a naphthyl group, TRPV1 antagonists with improved properties can be obtained.

Accordingly, the invention provides compounds of general formula (**I**) 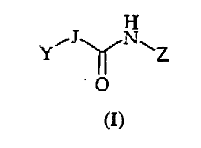 wherein:
J is a phenyl or a pyridyl ring;
Y is a naphthyl, quinolinyl or isoquinolinyl ring, linked to J at the *para* position to the carbonyl group and optionally substituted with a halogen atom;
Z is a phenyl or a pyridyl ring, optionally substituted with one or two R groups, which can be the same or different from one another and are selected from C₁-C₄ alkyl, preferably methyl, isopropyl or *tert*-butyl,
C₁-C₄ haloalkyl, preferably trifluomethyl, or halogen.

For the purposes of the present description, halogen means a halogen atom selected from fluorine, chlorine, bromine and iodine.

A first group of preferred compounds of the invention is that of formula (**Ia**) 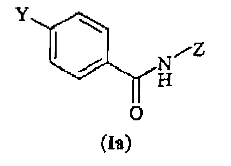 in which Y and Z are as defined above;

A second group of preferred compounds according to the invention is that of formula (**Ib**) 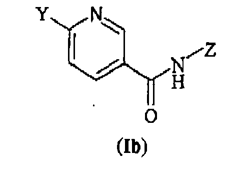 in which Y and Z are as defined above;

In the compounds of formula (**Ia**) and (**Ib**) Y is preferably naphthyl and Z is preferably a phenyl ring substituted at the para-position with an R group other than hydrogen.

Examples of preferred compounds according to the invention are the following:
N-(4-chlorophenyl)-4-(naphthalen-1-yl)benzamide (**1**)
N-(4-isopropylphenyl)-6-(naphthalen-1-yl)pyridine-3-carboxamide (**2**)
N-(3-methoxyphenyl)-4-(naphthalen-1-yl)benzamide (**3**)
N-(3-fluorophenyl)-4-(naphthaien-1-yl)benzamide (**4**)
4-(naphthalen-1-yl)-N-p-tolylbenzamide (**5**)
N-(4-isopropylphenyl)-4-(naphthalen-1-yl)benzamide (**6**)
N-(4-(trifluoromethyl)phenyl)-4-(naphthalen-1-yl)benzamide (**7**)
N-(4-fluorophenyl)-4-(naphthalen-1-yl)benzamide (**8**)
N-(4-bromophenyl)-4-(naphthalen-1-yl)benzamide (**9**)
N-(4-morpholinophenyl)-4-(naphthalen-1-yl)benzamide (**10**)
N-(4-tert-butylphenyl)-4-(naphthalen-1-yl)benzamide (**11**)
N-(6-chloropyridin-3-yl)-4-(naphthalen-1-yl)benzamide (**12**)
N-(2,4-dichlorophenyl)-4-(naphthalen-1-yl)benzamide (**13**)
N-(3-chlorophenyl)-4-(naphthalen-1-yl)benzamide (**14**)
N-(3-chloro-4-fluorophenyl)-4-(naphthalen-1-yl)benzamide (**15**)
N-(4-methoxyphenyl)-4-(naphthalen-1-yl)benzamide (**16**)
N-(3,4-dichlorophenyl)-4-(naphthalen-1-yl)benzamide (**17**)
N-(5-chloropyridin-2-yl)-4-(naphthalen-1-yl)benzamide (**18**)
N-(4-chlorophenyl)-4-(quinolin-3-yl)benzamide (**19**)
N-(4-chlorophenyl)-4-(isoquinolin-4-yl)benzamide (**20**)
N-(4-chlorophenyl)-6-(naphthalen-1-yl)pyridine-3-carboxamide (**21**)

Among them, particularly preferred are N-(4-chlorophenyl)-4-(naphthalen-1-yl)benzamide (**1**) 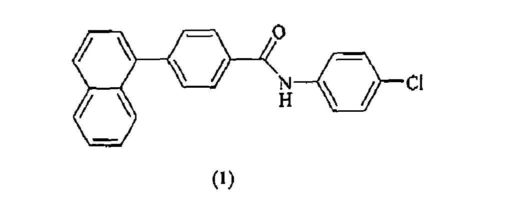 and N-(4-isopropylphenyl)-6-(naphthalen-1-yl)pyridine-3-carboxamide (**2**).

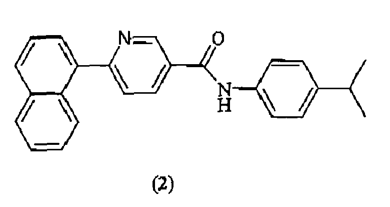

The compounds of formula (**I**) can be prepared by means of conventional methods, such as the reaction of a compound of formula (**II**) 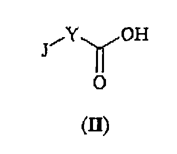 wherein J and Y are as defined above and the carboxyl group is activated as chloride
with a compound of formula (**III**)

Z-NH₂ **(III)**

wherein Z is as defined above.

Alternatively, the compounds of formula (**I**) can be obtained by Suzuki reaction² between a compound of formula (**IV**) 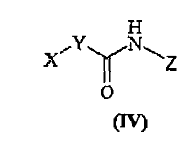 wherein Y and Z are as defined above and X is a halogen selected from iodine and bromine
and a boronic acid (**V**)

J-B(OH)₂ (**V**)

wherein J is as defined above.

The compounds of formula (**I**) modulate the vanilloid TRPV1 receptor; the preferred compounds show Kᵢ and IC₅₀ values of = 30-3000 nM and can be used for the preparation of pharmaceutical compositions for the treatment of inflammatory states, such as chronic pain and inflammatory hyperalgesia. These formulations can be prepared by conventional methods and excipients, such as those disclosed in Remington's Pharmaceutical Science Handbook, XVII ed. Mack Pub., N.Y., U.S.A..

The invention will be herein after illustrated by means of the following examples and of schemes 1, 2 and 3.

### EXAMPLES

### Materials and methods

All commercially available compounds were purchased from Aldrich and were used without further purification. Reaction courses were monitored by thin-layer chromatography on silica gel (precoated F₂₅₄ Merck plates), the spots were examined with UV light and visualized with aqueous KMnO₄. Flash chromatography was performed using Merck silica gel (230-240 mesh). ¹H-NMR spectra were recorded on a Varian 400 MHz spectrometer using TMS as internal standard. Mass spectra were obtained with a Waters-Micromass ZMD spectrometer. Melting points were determined on a Buchi-Tottoli apparatus and are uncorrected.

### Example 1 - N-(4-chlorophenyl)-4-(naphthalen-1-yl)benzenamide (1)

### Synthesis of N-(4-chlorophenyl)-4-(naphthalen-1-yl)benzenamide (1) (scheme 1)

To a solution of 4-(naphthalen-1-yl)benzoic acid³ (0.5 mmol, 125 mg) in toluene (10 ml), SOCl₂ (3 equiv., 1.5 mmol, 0.11 ml) was added at 0°C and the mixture was refluxed for 1 h. The solvent was evaporated and the residue was dissolved in 10 ml of CH₂Cl₂. 4-Chloroaniline (2 equiv., 1 mmol, 128 mg) was added at 0°C and the resulting mixture was stirred at room temperature for 20 h. The reaction mixture was diluted with 10 ml of CH₂Cl₂ and washed with HCl 5% (1 X 10 ml), H₂O (1 X 5 ml), a saturated sodium bicarbonate solution (1 X 5 ml), water (1 X 5 ml) and a saturated sodium chloride solution (1 X 5 ml). The organic phase was concentrated, and the residue was purified by column chromatography (silica gel, ethyl acetate/hexane 1/9) to give 150 mg of compound 1 as a white solid that was recrystallized from diethyl ether. Yield = 84%. Mp: (diethyl ether) 221°C. ¹H NMR (CDCl₃, 400 MHz) δ 7.36 (2H, m), 7.43 (2H, m), 7.58 (2H, m), 7.64 (4H, m), 7.83 (1H, d), 7.92 (3H, t), 8.00 (2H, d); [M⁺¹] 358.9/359.5 (C₂₃H₁₆ClNO requires 357.83).

### Synthesis of N-(4-chlorophenyl)-4-iodobenzenamide (scheme 2)

Commercially available 4-iodobenzoic acid (1.5 mmol, 395 mg) was dissolved in 5 ml of anhydrous DMF. EDCI (1.2 equiv., 1.8 mmol, 350 mg), HOBt (1.2 equiv., 1.8 mmol, 250 mg) and 4-chloroaniline (1.2 equiv., 1.8 mmol, 230 mg) were added sequentially at 0°C. The reaction mixture was stirred at room temperarture for 20 h. The solvent was evaporated under reduced pressure and the residue was dissolved in 40 ml of ethyl acetate. The organic phase was washed with 5% HCl (1 X 20 ml), H₂O (1 X 10 ml), a saturated sodium bicarbonate solution (1 X 10 ml), water (1 X 10 ml) and a saturated sodium chloride solution (1 X 10 ml). The organic layer was dried over sodium sulphate and concentrated. The crude solid was recrystallized from diethyl ether to give 450 mg of a white solid. Yield= 84%. ¹H NMR (CDCl₃, 400 MHz) δ 7.33 (2H, d, J = 6.4 Hz), 7.59 (4H, m), 7.76 (1H, bs), 7.85 (2H, d, J = 6.8 Hz); [M⁺¹] 358.3/359.2 (C₁₃H₉ClINO requires 357.57).

### Synthesis of N-(4-chlorophenyl)-4-(naphthalen-1-yl)benzenamide (1) (scheme 2)

A suspension of N-(4-chlorophenyl)-4-iodobenzamide (0.5 mmol, 180 mg), 1-naphthalene-boronic acid (1 mmol, 172 mg), anhydrous potassium carbonate (0.75 mmol, 104 mg) and toluene (10 ml) under argon was stirred at rt for 15 min. Catalytic tetrakis(triphenylphosphine)palladium(0) was added and the mixture was heated at 90°C for 16 h. The reaction mixture was cooled to 25°C, diluted with ethyl acetate (15 ml) and washed sequentially with a saturated sodium bicarbonate solution (1 X 20 ml), water (1 X 20 ml) and a saturated sodium chloride solution (1 X 20 ml). The organic phase was concentrated, and the residue was purified by column chromatography (silica gel, ethyl acetate/hexane 1/9) to give compound 1 in 80% yield.

### Example 2 - N-(4-isopropylphenyl)-6-(naphthalen-1-yl)pyridine-3-carboxamide (2)

### Synthesis of 6-bromo-N-(4-isopropylphenyl)pyridine-3-carboxamide (scheme 3)

The 6-bromopyridine-3-carboxylic acid⁴ (1.5 mmol, 300 mg) was dissolved in 5 ml of anhydrous DMF. EDCI (1.2 equiv., 1.8 mmol, 350 mg), HOBt (1.2 equiv., 1.8 mmol, 250 mg) and 4-chloroaniline (1.2 equiv. 1.8 mmol, 230 mg) were added sequentially at 0°C. The reaction mixture was stirred at room temperarture for 20 h. The solvent was evaporated under reduced pressure and the residue was dissolved in 40 ml of ethyl acetate. The organic phase was washed with 5% HCl (1 X 20 ml), H₂O (1 X 10 ml), saturated sodium bicarbonate solution (1 X 10 ml), water (1 X 10 ml) and a saturated sodium chloride solution (1 X 10 ml). The organic layer was dried over sodium sulphate and concentrated. The crude solid was recrystallized from diethyl ether to give 410 mg of a pale yellow solid. Yield= 85%. ¹H NMR (CDCl₃, 400 MHz) δ 1.24 (3H, s), 1.25 (3H, s), 2.91 (1H, m), 7.23 (2H, d, J = 8.8 Hz), 7.46 (1H, d), 7.52 (1H, d, J = 8.8 Hz), 7.77 (1H, bs), 8.16 (1H, m), 8.85 (1H, s); [M⁺¹] 320.3/322.1 (C₁₅H₁₅BrN₂O requires 319.2).

### Synthesis of N-(4-isopropylphenyl)-6-(naphthalen-1-yl)pyridine-3-carboxamide (2) (scheme 3)

A suspension of 6-bromo-N-(4-isopropylphenyl)pyridine-3-carboxamide (0.5 mmol, 160 mg), 1-naphthalene-boronic acid (1 mmol, 172 mg), anhydrous potassium carbonate (0.75 mmol, 104 mg) and toluene (10 ml) under argon was stirred at rt for 15 min. Catalytic tetrakis(triphenylphosphine)palladium(0) was added and the mixture was heated at 90°C for 16 h. The reaction mixture was cooled to 25°C, diluted with ethyl acetate (15 ml) and washed sequentially with a saturated sodium bicarbonate solution (1 X 20 ml), water (1 X 20 ml) and saturated sodium chloride solution (1 X 20 ml). The organic phase was concentrated, and the residue was purified by column chromatography (silica gel, ethyl acetate/hexane 1/9) to give 150 mg of compound 2 as a pale yellow solid. Yield = 80%. Mp: (hexanes) 132-134°C, ¹H NMR (CDCl₃, 400 MHz) δ 1.25 (3H, s), 1,27 (3H, s), 2.96 (1H, m), 7.27 (2H, d), 7.52 (4H, m), 7.64 (2H, d), 7.72 (1H, d), 7.90 (2H, d), 8.00 (1H, d), 8.38 (1H, bs), 8.40 (1H, d), 9.39 (1H, s); [M⁺¹] 367.1 (C₂₅H₂₂N₂O requires 366.45). 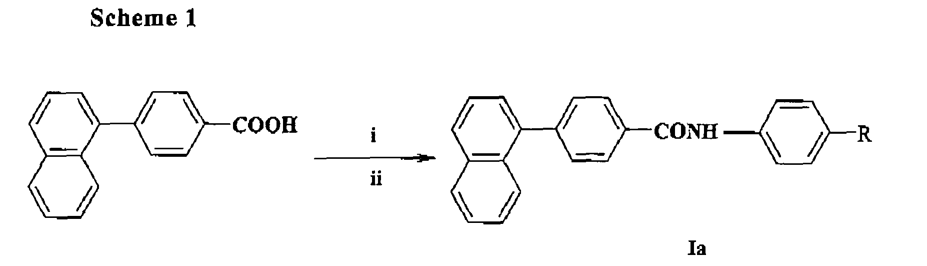 **Reagents: i)** SOCl₂, toluene, rfx, 1h; **ii)** 4-substituted aniline, TEA, CH₂Cl₂, rt, 20 h.

### Compound 1: R = Cl

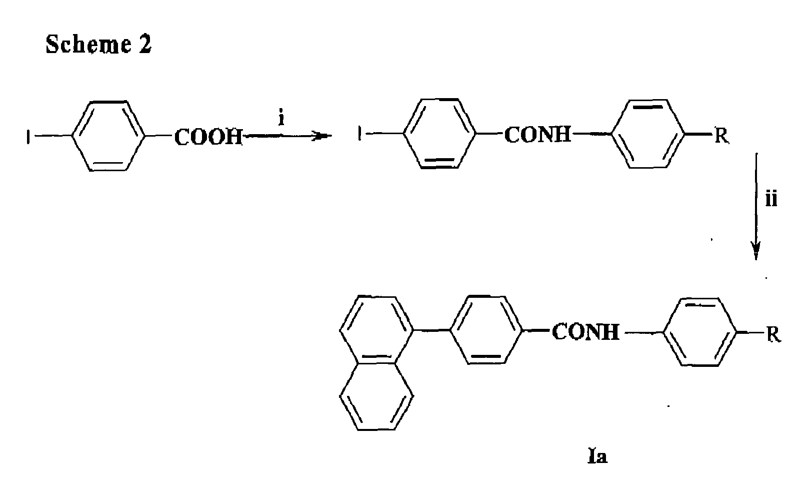

**Reagents: i)** EDCI, HOBt, 4-substitued aniline, DMF, 20h, rt; ii) 1-naphthtalene boronic acid, an. K₂CO₃, Pd(PPh₃)₄, toluene, 90°C, 16 h. 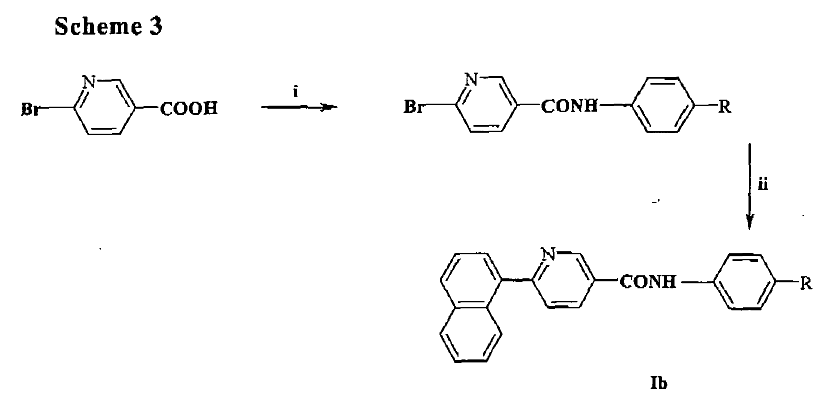 **Reagents: i)** EDCI, HOBt, 4-substitued aniline, DMF, 20b, rt; ii) 1-naphthtalene boronic acid, an. K₂CO₃, Pd(PPh₃)₄, toluene, 90°C, 16 h.

### Cobnpound 2: R = CH-(CH₃)₂

### Biological Assay

Newborn and adult Sprague-Dawley rats (-250 g) were used (Harlam, Italy). All experiments complied with the national guidelines and were approved by the regional ethics committee.

### Radioligand binding assay

Male Sprague-Dawley rats with body weight between 250 to 350 g at the time for testing were used. For binding assays rats were sacrificed by decapitation under anesthesia and the spinal cord was removed and disrupted using a Polytron tissue homogenizer in ice cold buffer containing 5 mM KCI, 5.8 mM NaCI, 0.75 mM CaCl₂, 2 mM MgCl₂, 320 mM sucrose, 10 mM Hepes, pH 8.6.⁵ The homogenized tissue was centrifuged at 1000 x g for 10 min at 4°C and the surnatant was centrifuged again at 35000 x g for 30 min at 4°C (Beckman Avanti J25). The pellet was resuspended in the same buffer as described above and used in binding experiments. In saturation experiments, 150 µg protein/sample from membrane suspensions were incubated with [³H]-Resiniferatoxin ([³H]-RTX) (0.003-3 nM) in the assay buffer containing 0.25 mg/ml fatty acid-free bovine serum albumin at 37°C for 60 min. In competition experiments, the membranes were incubated at 37°C for 60 min with [³H]RTX (0.4 nM) and increasing concentrations of examined compounds in the range from 0.1 nM to 3 µM.

Non specific binding was defined in the presence of 1 µM RTX. After incubation the reaction mixture was cooled at 0°C and incubated with bovine α1-acid glycoprotein (200 µg per tube) for 15 min to reduce non-specific RTX binding. Membrane-bound RTX was separated from the free through the centrifugation of the samples at 18500 x g for 15 min. The tip of the microcentrifuge tube containing the pellet was cut off and the radioactivity was determined by scintillation counting (Packard 2500 TR). The protein concentration was determined according to a Bio-Rad method with bovine serum albumin as a standard reference (Bradford, 1976). Saturation and competition studies were analyzed with the program Ligand.⁶

### Ca²⁺ fluorescence measurements in cultured rat trigeminal ganglia,

Newborn rats (2 days old) were terminally anaesthetized and decapitated. Trigeminal ganglia were removed and rapidly placed in a cold phosphate buffered solution (PBS) before being transferred to collagenase/dispase (1 mg/ml dissolved in Ca²⁺- Mg²⁺-free PBS) for 35 min at 37°C.⁷ After the enzymatic treatment the ganglia were rinsed three times with Ca²⁺- Mg²⁺-free PBS and then placed in 2 ml of cold DMEM supplemented with 10 % fetal bovine serum (FBS, heat inactivated), 2 mM L-glutamine, 100 µ/ml penicillin and 100 µg/ml streptomycin. The ganglia were dissociated into single cells by several passages through a series of syringe needles (23G down to 25G). Finally, the medium and ganglia cells were sieved through a 40 µm filter to remove debris and topped up with 8 ml of DMEM medium and centrifuged (200 x g for 5 min). The final cell pellet was re-suspended in DMEM medium [supplemented with 100 ng/ml mouse Nerve Growth Factor (mouse-NGF-7S) and cytosine-β-D-arabinofuranoside free base (ARA-C) 2.5 µM]. The cells were plated on poly-L-lysine (8.3 µM)- and laminin (5 µM)- coated 25 mm glass cover slips and kept for 5 to 8 days at 37°C in a humidified incubator gassed with 5% CO₂ and air. Plated neurons were loaded with Fura-2-AM-ester (3 µM) in Ca²⁺ buffer solution of the following composition (mM): CaCl₂ 1.4, KCl 5.4, MgSO₄ 0.4, NaCl 135, D-glucose 5, HEPES 10 with BSA (0.1%), at pH 7.4, for 40 min at 37°C. The plated neurons were then washed twice with the Ca²⁺ buffer solution and transferred to a chamber on the stage of Nikon eclipse TE300 microscope. Fura-2-AM-ester was excited at 340 nM and 380 nM to indicate relative [Ca²⁺]ᵢ changes by the F₃₄₀/F₃₈₀ ratio recorded with a dynamic image analysis system (Laboratory Automation 2.0, RCS, Florence, Italy). After transferring the plated neurons to the chamber, they were allowed (at least 10 min) to attain a stable fluorescence before beginning the experiment. A calibration curve was performed using buffer containing Fura-2-AM-ester and determinant concentrations of free Ca²⁺. This curve was then used to convert the data obtained from the F₃₄₀/F₃₈₀ ratio to [Ca²⁺]ᵢ (nM).⁸ The effects of pretreatments with capsazepine (CPZ), SB366791 and compounds (I) on the increase in [Ca²⁺]ᵢ produced by 0.1 µM capsaicin were studied.

### Wiping test in rats

Rats were acclimatized for 1h in a 30 x 30 x 30-cm Plexiglas chamber before the intraperitoneal injection of the TRPV1 ligands or their vehicles (7% DMSO and 7% Tween80). Injections were made in the lower right ventral quadrant of the abdomen 60 and 120 min prior intraocular application of capsaicin. Intraocular application of capsaicin (3 µg/5 µl/eye in 0.5% ethanol/physiologic solution) or vehicle was carried out with a micropipette and the number of front paw eye wipes was counted over a 1-min period. Data from the first-minute interval were used because capsaicin-evoked eye wiping behavior occurred only during the first minute of observation. Each group consisted of 8 to 10 animals,

### Drugs and Reagents

Drugs and reagents were obtained from the indicated companies: [³H]-Resiniferatoxin (Perkin Elmer, Boston, MA), SB-366791 (Tocris, UK), capsaicin, capsazepine, ionomycin, laminin, poly-L-lysine, substance P (Sigma, Italy); mouse NGF-7S and collagenase/dispase (Roche Diagnostics, Italy); Dulbecco's Modified Eagle's medium (DMEM), foetal bovine serum (FBS) heat inactivated, L-glutamine (200 mM), penicillin/streptomycin (10,000 IU/ml ± 10,000 UG/ml), (Gibco, Italy); Fura-2-AM-ester (Società Italiana Chimici, Italy). The stock concentrations of capsaicin (10 mM), capsazepine (10 mM) SB-366791 (1 mM) and compounds I were prepared in 50% DMSO and 50% Tween 80, Fura-2-AM-ester and ionomycin were dissolved in 100% DMSO. All other drugs were dissolved in distilled water. The appropriate dilutions were then made in Krebs buffer solution.

### Results

### Radioligand Binding assay

The saturation curve of [³H]-RTX to TRPV1 expressed in rat spinal cord showed a K_{D} value of 0.21 (0.16-0.27) and a Bₘₐₓ value of 57 (53-62) fmol/mg protein. The Scatchard plot was essentially linear and computer analysis of the data indicated that only one class of high affinity binding sites was present. Competition binding experiments of [³H]-RTX revealed that compounds **1, 2** and the reference SB-366791 had a Kᵢ value of 120 (103-140), 34 (26-45) and 36 (30-43) nM respectively.

### Ca²⁺ fluorescence

Capasaicin (0.1 µM) caused an increase in [Ca²⁺] in the vast majority (95%) of rat trigeminal neurons cells, that therefore were identified as TRPV1 expressing neurons. IC₅₀ values of 1 and 2 inhibiting capsaicin-evoked [Ca²⁺]ᵢ mobilitazion were 91 (37-226) nM and 190 (180-200) nM, respectively. The reference TRPV1 antagonists, capsazepine and SB-366791, inhibited the capsaicin response with an IC₅₀ of 948 (676-1330) nM and 8.7 (3.4-17.3) nM, respectively. The results are expressed as Mean and 95% fiducial limits.

### Eye Wipe Test in Rats

A dose-dependent inhibition of capsaicin-induced eye wipes in rat was studied at 60 min from the administration of compound 1 and two reference compounds: capsazepine, SB-366791. All the antagonists were tested at the doses of 0.3, 1 and 3 mg/kg, administered intraperitoneally.

The potency of the tested compounds is described by using the dose of antagonist that produces 50% of the maximal inhibitory effect observed with the highest dose/concentration of the above mentioned chemicals (ED₅₀). ED₅₀ values (and respective standard errors, SEM) were calculated by standard linear regression analysis of the log dose/concentration-response curves.

ED₅₀ values for capsazepine, SB-366791 and 1 were 1.55 ± 0.39, 0.71 ± 0.20 and 0.45 ± 0.14 mg/kg, respectively.

### REFERENCES

1. Bakthavatchalam, R.; Blum, C.A.; Brielmann, H.; Darrow, J.W.; De Lombaert, S.; Yoon, T.; Zheug, X. Neurogen Corporation WO 2004/056774, 170pp.
2. Shieh, W.C.; Carlson, J.A.J. Org. Chem. 1992, 57, 379-381.
3. Zhang, J.; Assodi, J.; Charter, C.; L'hermite, N.; Weston, J. Tetrahedron Lett. 2001, 42, 6683-6686.
4. Meier, P.; Legraverant, S.; Mueller, S.; Schaub, J. Synthesis 2003, 4, 551-554.
5. a) Szallasi A. and Blunberg P.M. Neurosciences 1992, 8, 368. b) Szallasi A. and Blunberg P.M. Naunyn Schmiedeberg's Arch Pharmacol. 1993, 347, 84-91.
6. Munson, P.J.; Rodbard, D. Anal. Biochem. 1980, 107, 220-239.
7. Rigoni, M.; Trevisani, M.; Gazzieri, D.; Nadaletto, R.; Tognetto, M.; Creminon, C.; Davis, J.B.; Campi, B.; Amatesi, S.; Geppetti, P.; Harrison, S. Br. J. Pharmacol. 2003, 138, 977-985.
8. Kudo, Y.; Ozaki, K.; Miyakawa, A.; Amano, T.; Ogura, A. Jap. J. Pharmacol. 1986, 41, 345-351.

## Claims

1. Compounds of general formula (**I**) 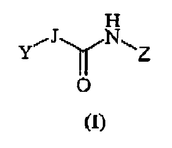 wherein:
J is a phenyl or a pyridyl group;
Y is a naphthyl, quinolinyl or isoquinolinyl group, linked to J at the *para* position to the carbonyl group and optionally substituted with a halogen atom;
Z is a phenyl or a piridyl group, optionally substituted with one or two
R groups, which can be the same or different from one another, and are selected from C₁-C₄ alkyl, C₁-C₄ haloalkyl, or halogen.

2. Compounds according to claim 1 wherein the R groups are selected from methyl, isopropyl or *tert*-butyl.

3. Compounds according to claim 1 wherein the R groups are trifluomethyl.

4. A compound according to claim 1 of formula (**Ia**) 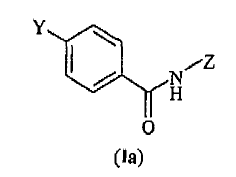 in which Y and Z are as defined in claim 1.

5. A compound according to claim 1 of formula (**Ib**) 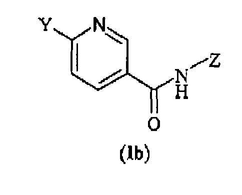 in which Y and Z are as defined in claim 1.

6. A compound according to claim 4 or 5 wherein Y is naphthyl and Z is a phenyl ring substituted at the para-position with one R group as defined in claim 1.

7. A compound according to claim 1 which is selected from:
N-(4-chlorophenyl)-4-(naphthalen-1-yl)benzamide;
N-(4-isopropylphenyl)-6-(naphthalen-1-y1)pyridine-3-carboxamide;
N-(3-methoxyphenyl)-4-(naphthalen-1-yl)benzamide;
N-(3-fluorophenyl)-4-(naphthalen-1-yl)benzamide;
4-(naphthalen-1-yl)-N-p-tolylbenzamide;
N-(4-isopropylphenyl)-4-(naphthalen-1-yl)benzamide;
N-(4-(trifluoromethyl)phenyl)-4-(naphthalen-1-yl)benzamide;
N-(4-fluorophenyl)-4-(naphthalen-1-yl)benzamide;
N-(4-bromophenyl)-4-(naphthalen-1-yl)benzamide;
N-(4-morpholinophenyl)-4-(naphthalen-1-yl)benzamide;
N-(4-tert-butylphenyl)-4-(naphthalen-1-yl)benzamide;
N-(6-chloropyridin-3-yl)-4-(naphthalen-1-yl)benzamide;
N-(2,4-dichlorophenyl)-4-(naphthalen-1-yl)benzamide;
N-(3-chlorophenyl)-4-(naphthalen-1-yl)benzamide;
N-(3-chloro-4-fluorophenyl)-4-(naphthalen-1-yl)benzamide;
N-(4-methoxyphenyl)-4-(naphthalen-1-yl)benzamide;
N-(3,4-dichlorophenyl)-4-(naphthalen-1-yl)benzamide;
N-(5-chloropyridin-2-yl)-4-(naphthalen- I -yl)benzamide;
N-(4-chlorophenyl)-4-(quinolin-3-yl)benzamide;
N-(4-chlorophenyl)-4-(isoquinolin-4-yl)benzamide;
N-(4-chlorophenyl)-6-(naphthalen-1-yl)pyridine-3-carboxamide.

8. Compounds of any one of claims 1 to 6 for use as medicaments.

9. Use of the compounds of any one of claims 1 to 7 for the preparation of medicaments for the treatment of inflammatory diseases and urological disorders.

10. Use according to claim 8 wherein the inflammatory disease is neuropathic pain.

11. Pharmaceutical compositions containing a compound of any one of claims 1 to 4 in ad mixture with pharmaceutically acceptable carriers and/or excipients.
